(19) Europäisches Patentamt
European Patent Office
Office européen des brevets

(11)  EP 3 432 989 B1

(12)  **FASCICULE DE BREVET EUROPEEN**

(45) Date de publication et mention de la délivrance du brevet:
**10.01.2024  Bulletin 2024/02**

(51) Classification Internationale des Brevets (IPC):
*A61Q 5/00* $^{(2006.01)}$   *A61Q 19/00* $^{(2006.01)}$
*A61K 8/19* $^{(2006.01)}$   *A61K 33/00* $^{(2006.01)}$
*A61K 35/08* $^{(2015.01)}$   *C02F 1/44* $^{(2023.01)}$
*A61P 17/00* $^{(2006.01)}$

(21) Numéro de dépôt: **17717775.5**

(22) Date de dépôt: **15.03.2017**

(52) Classification Coopérative des Brevets (CPC):
**A61K 8/19; A61K 33/00; A61K 35/08; A61P 17/00; A61Q 5/00; A61Q 19/00; A61Q 19/005; A61Q 19/007; C02F 1/441;** C02F 2103/026

(86) Numéro de dépôt international:
**PCT/FR2017/050601**

(87) Numéro de publication internationale:
**WO 2017/162957 (28.09.2017 Gazette 2017/39)**

(54) **EAU DE SOURCE OU MINÉRALE CONCENTRÉE ENRICHIE EN ACIDE ORTHOSILICIQUE NATUREL**

QUELL- ODER MINERALWASSER ANGEREICHERT MIT NATÜRLICHEM ORTHOKIESELÄURE

SPRING OR MINERAL WATER ENRICHED WITH NATURAL ORTHOSILICIC ACID

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **22.03.2016  FR 1652455**

(43) Date de publication de la demande:
**30.01.2019  Bulletin 2019/05**

(73) Titulaire: **Societe Des Eaux De Source De Treignac 19260 Treignac (FR)**

(72) Inventeurs:
 • **SAINTE-LAUDY, Jean**
   **56890 Saint-Avé (FR)**
 • **PONS, Annick**
   **75010 Paris (FR)**
 • **REDZINIAK, Gérard**
   **92160 Antony (FR)**
 • **KITA, Waldemar**
   **10180 Uccle (BE)**
 • **CHERON, Michel**
   **75008 Paris (FR)**

(74) Mandataire: **Gevers & Orès**
   **Immeuble le Palatin 2**
   **3 Cours du Triangle**
   **CS 80165**
   **92939 Paris La Défense Cedex (FR)**

(56) Documents cités:
**WO-A1-2011/009950   US-B1- 6 335 457**

 • **GLYCAN SHOP-GLYCAN GROUP:** "Le Silicium Organique G5 Glycan", INTERNET CITATION, 9 avril 2009 (2009-04-09), pages 1-2, XP002524736, Extrait de l'Internet: URL:http://www.le-silicium-organique.com/boutique/115-silicium-organique-g5-1500mg-1 .html [extrait le 2009-04-09]
 • **BAREL A ET AL:** "Effect of oral intake of choline-stabilized orthosilicic acid on skin, nails and hair in women with photodamaged skin", ARCHIVES OF DERMATOLOGICAL RESEARCH ; FOUNDED IN 1869 AS ARCHIV FÜR DERMATOLOGIE UND SYPHILIS, SPRINGER, BERLIN, DE, vol. 297, no. 4, 1 octobre 2005 (2005-10-01), pages 147-153, XP019341162, ISSN: 1432-069X, DOI: 10.1007/S00403-005-0584-6

- CALOMME M R ET AL: "SUPPLEMENTATION OF CALVES WITH STABILIZED ORTHOSILICIC ACID EFFECT ON THE SI, CA, MG, AND P CONCENTRATIONS IN SERUM AND THE COLLAGEN CONCENTRATION IN SKIN AND CARTILAGE", BIOLOGICAL TRACE ELEMENT RESEARCH, HUMANA PRESS, CLIFTON, NJ, US, vol. 56, 1 février 1997 (1997-02-01), pages 153-165, XP000925562, ISSN: 0163-4984

**Description**

La présente invention concerne une eau (eau minérale ou eau de source) concentrée ayant conservé son équilibre ionique d'origine et enrichie en acide orthosilicique naturel, un procédé de production d'une telle eau et une composition diététique, cosmétique, dermatologique ou pharmaceutique ainsi qu'un complément nutritionnel la comprenant.

L'invention concerne également l'utilisation d'une eau (eau minérale ou eau de source) concentré enrichie en acide orthosilicique naturel pour régénérer et/ou renforcer la peau, les cheveux et les ongles ; et/ou pour hydrater et prévenir la déshydratation de la peau.

L'invention concerne, en outre, une eau concentrée (eau minérale ou eau de source) enrichie en acide orthosilicique naturel destinée à être utilisée comme médicament, notamment dans le traitement et/ou la prévention des maladies neurodégénératives, en particulier la maladie d'Alzheimer, le traitement des peaux sensibles ; le traitement et/ou la prévention des pathologies inflammatoires de la peau ; la prévention des maladies cardiovasculaires et/ou de l'athérosclérose.

L'invention s'étend également à un dispositif renfermant ou contenant une eau concentrée selon l'invention ou une composition diététique, cosmétique, dermatologique ou pharmaceutique comprenant une eau concentré selon l'invention.

Le silicium est, après l'oxygène, le second élément le plus abondant sur terre, constituant 28% de la masse de la croûte terrestre. Il est présent dans la plupart des roches minérales, des sables et des argiles.

En tant qu'oligo-élément, il se charge de nombreuses fonctions dans l'organisme, la plus importante étant sans conteste la formation du tissu conjonctif, ce qui explique son influence sur la peau, les cheveux et les ongles. Il a été montré que le silicium améliore l'élasticité et l'hydratation des tissus dont la peau. Grâce à ses propriétés anti-inflammatoires, le silicium peut protéger la peau contre les infections et/ou inflammations.

Par son action sur le collagène et les fibres élastiques, il a été démontré que le silicium joue un rôle clé dans la-physiologie des-tissus conjonctifs.

Le silicium est impliqué dans la fixation du calcium ($Ca^{2+}$) au niveau du squelette et peut donc intervenir dans la formation de la matrice osseuse et la minéralisation de l'os.

Le silicium participe au fonctionnement du système immunitaire. Le thymus qui en contient des quantités importantes est l'organe où se "programment" les lymphocytes T.

L'aorte est le tissu qui contient le plus de silicium avec la peau et le thymus. La diminution du silicium dans les parois artérielles est corrélée avec la fragmentation de l'élastine et à la perte de son élasticité.

Le silicium peut, en outre, intervenir dans le traitement et/ou la prévention des maladies neurodégénératives, en particulier la maladie d'Alzheimer en raison de sa capacité à réduire l'absorption d'aluminium dans le système digestif et à provoquer l'excrétion rénale de l'aluminium.

Aujourd'hui, les eaux et les boissons pouvant apporter des propriétés favorables à la santé sont de plus en plus intéressantes pour les consommateurs, car elles peuvent aider, dans certains cas, à prévenir les problèmes de santé à long terme. Quand ils ont le choix, les consommateurs préfèrent toujours les produits naturels avec peu ou pas de calories, comme l'eau.

Les eaux minérales sont caractérisées par des propriétés biologiques avantageuses pour l'organisme dues à leur composition physico-chimique et à l'équilibre naturel subtil de leurs constituants. L'activité biologique des eaux minérales dépend étroitement de leur environnement physico-chimique et de la présence éventuelle de composants interférents (principalement les ions divalents et les bicarbonates).

Les eaux peuvent ainsi constituer un excellent vecteur pour l'apport des minéraux en général. Parmi ces minéraux et oligoéléments, le silicium occupe une place à part car il constitue un paramètre du vieillissement. Un apport complémentaire en silicium, de préférence naturel, est donc souhaitable.

Le silicium n'existe pas dans la nature à l'état de corps pur mais sous forme de composés oxydés comme, par exemple, sous la forme de silice ($SiO_2$). Dans l'eau, la silice subit une hydrolyse qui aboutit à la formation d'acide orthosilicique $Si(OH)_4$, faiblement acide ($pKa_1=9,84$ et $pKa_2=13,2$ à 25°C) et stable à pH neutre, selon la réaction suivante :

$$SiO_2 + 2H_2O \rightarrow Si(OH)_4$$

Ainsi, dans l'eau, dans les conditions normales de température (une température de 20 ± 5°C) et de pression (une atmosphère), le silicium existe essentiellement sous forme d'acide orthosilicique ($Si(OH)_4$).

Divers essais ont concerné la production *in vitro* d'eau enrichie en acide orthosilicique mais ces procédés nécessitent des traitements physiques sévères (tels que le passage à haute température) susceptibles d'altérer l'équilibre naturel de l'eau d'origine.

Ainsi, la forme chimique dans laquelle est retrouvé le silicium naturel est l'acide orthosilicique qui a l'avantage d'être la forme la plus assimilable pour l'homme.

Les propriétés de condensation (polymérisation) l'acide orthosilicique dépendent de la non salification des groupements hydroxyles du silicium organique, par exemple, mais également de la présence la plus faible possible de composants interférants (principalement les ions divalents et les bicarbonates).

La solubilité de l'acide orthosilicique $Si(OH)_4$ dans l'eau à pH neutre et à 25°C est d'environ 120 mg/l et augmente significativement avec le pH, particulièrement au-dessus de 9. Cette solubilité est classiquement exprimée en milligrammes de $SiO_2$ par litre d'eau avec le facteur de conversion étant : 1 mg/l of $SiO_2$ = 1.6 mg/l $Si(OH)_4$. Cette convention est celle adoptée dans la suite de la description.

Comme indiqué, l'acide orthosilicique est un diacide faible ($pKa_1$=9,84 et $pKa_2$=13,2 à 25°C) qui est sous forme non dissocié à un pH neutre ou acide. L'acide orthosilicique a une bonne biodisponibilité. Le pourcentage d'acide orthosilicique ingéré et/ou absorbé par la muqueuse intestinale humaine étant de l'ordre de 50%. Toutefois, sa concentration dépend du type d'eau. L'acide orthosilicique est le constituant principal de certaines eaux très faiblement minéralisées, comme par exemple l'eau de Treignac.

Les eaux faiblement (le résidu sec inférieur à 500 mg/1) ou moyennement minéralisées (le résidu sec entre 500 et 1500 mg/l) contenant 30 mg/l ou plus d'acide orthosilicique sont rares. Les eaux faiblement minéralisées dont le résidu sec est inférieur à 30 mg/l contiennent généralement moins de 10 mg/l de silice. Les eaux fortement minéralisées (le résidu sec supérieur à 1500 mg/l) peuvent éventuellement contenir 30 mg/l ou plus d'acide orthosilicique, mais la teneur des autres principaux minéraux comme le magnésium, le calcium, le sodium, et les bicarbonates sera également élevée. Dans le cas d'un apport ciblé en silicium, il n'est pas nécessaire ni souhaité d'avoir une teneur trop importante en d'autres minéraux potentiellement interférents.

Le brevet EP2456327 décrit un procédé de production d'une eau enrichie en acide orthosilicique naturel qui comprend une teneur en acide orthosilicique comprise entre 45 et 120 mg/l de SiO2, et un résidu sec compris entre 280 et 1100 mg/l.

Les eaux minérales ou de source faiblement minéralisées (un résidu sec inférieur à 500 mg/l) ayant une teneur accrue (enrichie) en silicium présentent donc un intérêt tout particulier.

Il existe donc un réel besoin d'une eau faiblement minéralisée ayant une teneur accrue (enrichie) en une forme stable et naturelle de silicium tout en conservant son équilibre ionique d'origine.

En particulier, il existe un réel besoin d'une eau faiblement minéralisée ayant une teneur accrue (enrichie) en une forme naturelle de silicium ayant une bonne biodisponibilité, qui soit soluble, et chimiquement stable aux différents pH physiologiques c'est-à-dire en milieu légèrement acide, neutre ou légèrement alcalin.

Dans le présent exposé, l'expression « une forme naturelle de silicium » désigne une forme de silicium soluble et naturellement présente dans les eaux, c'est-à-dire l'acide orthosilicique.

La présente invention a précisément pour but de répondre à ces besoins en fournissant une eau de source ou minérale concentrée caractérisée en ce qu'elle présente un résidu sec inférieur à 195 mg/l à 180°C et une teneur en acide orthosilicique exprimée en ($SiO_2$) d'au moins 40 mg/l, ladite eau concentrée ayant conservé son équilibre ionique d'origine.

La teneur en acide orthosilicique est classiquement exprimée en milligrammes de $SiO_2$ par litre d'eau. Cette convention est celle adoptée dans la présente description.

L'eau de source ou minérale concentrée de l'invention est une eau faiblement minéralisée. Son résidu sec à 180°C est inférieur à 195 mg/l, de préférence, compris entre 20 et 190 mg/l, bornes incluses.

Le résidu sec représente le taux des minéraux recueillis après l'évaporation d'un litre d'eau soumis à une température de180 °C. Le résidu sec est un indicateur montrant si une eau est peu ou fortement minéralisée. Par exemple,

- Un résidu sec supérieur à 1500 mg/l correspond à une eau dite riche en minéraux ;
- Un résidu sec compris entre 500 et 1500 mg/l, l'eau est considérée comme moyennement minéralisée ;
- Un résidu sec compris entre 50 et 500 mg/l, correspond à une eau faiblement minéralisée ; et
- Un résidu sec inférieur à 50 mg/l, l'eau est considérée comme très faiblement minéralisée.

Parmi les eaux faiblement minéralisées, certaines ont un résidu sec compris entre 15 et 50mg/l. Par exemple, le résidu sec de l'eau de Treignac est de 20mg/l. De préférence, l'eau à partir de laquelle l'eau concentrée de l'invention est produite, est l'eau de Treignac.

Les résidus secs à 180°C des eaux minérales sont compris entre 18-20mg/l pour les moins minéralisées à 2600 mg/l pour les plus minéralisées.

La teneur en acide orthosilicique exprimée en $SiO_2$ d'une eau selon l'invention est, de préférence, comprise entre 40 et 60 mg/l, bornes incluses.

Il est à noter que l'eau concentrée de l'invention est une eau sensiblement enrichie en acide orthosilicique qui est en une forme de silicium soluble et naturellement présente dans les eaux, sans modifier les rapports ioniques.

En effet, la teneur en acide orthosilicique de l'eau concentrée de l'invention est plus de 8 fois supérieure à celle de l'eau utilisée pour préparer l'eau concentrée de l'invention.

L'acide orthosilicique contenu dans l'eau concentrée de l'invention est d'origine naturelle, provenant de l'eau utilisée pour sa préparation et non d'une source exogène de synthèse. Cet élément est fondamental afin de conserver les propriétés physico-chimiques de l'acide orthosilicique naturellement dissout, par exemple, à partir du sous-sol granitique et métamorphique caractéristique de l'impluvium concerné.

L'eau concentrée de l'invention a donc une forte teneur en acide orthosilicique et une teneur à l'état de traces d'ions potentiellement interférant comme par exemple le calcium, le sodium et le bicarbonate. Cette caractéristique peut présenter des avantages, notamment dans les domaines cosmétiques et/ou dermatologiques dans lesquels l'application de l'eau concentrée de l'invention à la surface de la peau forme un film homogène dépourvu de cristaux. L'effet filmogène et l'absence de cristaux dans le film formé par l'eau concentrée de l'invention a pour conséquence, par exemple, une meilleure tolérance cutanée, une meilleure hydratation ainsi qu'un effet apaisant et/ou anti-inflammatoire amélioré au niveau cutané.

La faible teneur en ions potentiellement interférant comme par exemple le calcium, le sodium et le bicarbonate, est également particulièrement intéressant lorsque l'eau concentrée est destinée à une utilisation dans une composition diététique ou dans un complément nutritionnel. En effet, dans de telles utilisations, il est souhaitable que l'absorption de l'acide orthosilicique au niveau de la muqueuse intestinale ne soit pas freinée par la présence de certains ions interférents.

Bien que le procédé de production de l'eau concentrée selon l'invention ait respecté l'équilibre ionique d'origine, le résidu sec reste faible et, par voie de conséquence, le teneur en ions potentiellement interférents tels que les ions divalents et particulièrement, pour l'eau de Treignac, le calcium et le magnésium et les bicarbonates, ces derniers se retrouvant dans l'eau concentrée à une concentration particulièrement faible.

La teneur en bicarbonate ($HCO_3^-$) de l'eau concentrée est inférieure ou égale à 30 mg/l, de préférence inférieure ou égale à 25 mg/l.

La teneur en calcium ($Ca^{2+}$) de l'eau de l'invention est inférieure ou égale à 8,5 mg/l, de préférence inférieure ou égale à 7 mg/l.

La présence de faibles concentrations d'ions divalents (magnésium, calcium sous forme de bicarbonates) est favorable à la polymérisation de l'acide orthosilicique, cette polymérisation nécessitant l'existence de groupement hydroxyles libres. Par exemple, dans l'eau de Treignac naturelle (avant concentration), les concentrations molaires de calcium, de magnésium et d'acide orthosilicique sont respectivement de 0,03 mM, 0,02 mM et 0,11 mM. Dans l'eau concentrée de Treignac les concentrations molaires de calcium, de magnésium et d'acide orthosilicique sont respectivement de 0,21 mM, 0,14 mM et 0,85 mM. Ceci correspond à 0,05 mM de cations divalents pour l'eau naturelle non concentrée et à 0,35 mM de cations divalents pour l'eau concentrée. Sachant que l'acide orthosilicique possède 4 groupes hydroxyle, le rapport acide orthosilicique/ cations divalents est de 4,4 pour l'eau naturelle et de 5 pour l'eau concentrée. Cela explique l'effet filmogène de l'eau concentrée de l'invention.

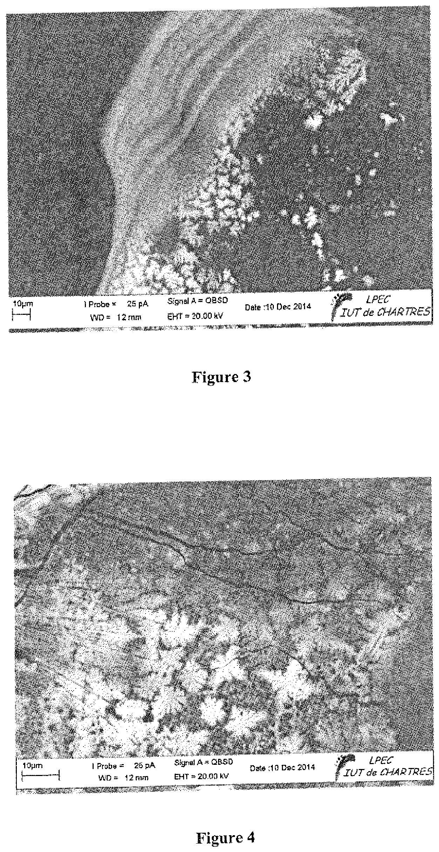

Le même calcul avec l'eau d'Evian donne un rapport de 0,15 pour l'eau d'Evian naturelle et 3,85 pour l'eau d'Evian concentrée, ce qui explique, au moins pour l'eau naturelle, un effet filmogène moins bon et la formation de cristaux potentiellement agressifs comme montré en figure 4.

A l'inverse, la synergie calcium-silice dissoute préside à la formation des os.

La teneur en sodium ($Na^+$) est également faible, c'est-à-dire inférieure ou égale à 18 mg/l, de préférence inférieure ou égale à 16 mg/l.

L'eau de source ou minérale concentrée de l'invention a un pH compris entre 6,0 et 7,5. De préférence, le pH est compris entre 6,0 et 7,2.

L'invention a également pour objet un procédé de production d'eau de source ou minérale concentrée selon l'invention, caractérisé en ce qu'une eau ayant un résidu sec à 180°C inférieur à 50 mg/l subit une osmose inverse de façon à obtenir une eau concentrée présentant un résidu sec inférieur à 195 mg/l à 180°C et une teneur en acide orthosilicique exprimée en ($SiO_2$) d'au moins 40 mg/l.

Le procédé de production d'eau concentrée selon l'invention a pour but de concentrer une eau minérale ou une eau de source en une seule étape, par osmose inverse, sans perturber l'équilibre ionique naturel de l'eau d'origine (eau minérale ou eau de source) et d'obtenir ainsi une eau faiblement minéralisée, de composition bien inférieure aux règles de potabilité et enrichie en acide orthosilicique, idéalement > 50mg/l, sachant que la limite de solubilité de cet acide à pH neutre ou légèrement acide est de l'ordre de 120-140mg/l.

De manière inattendue, le procédé de production de l'eau de source ou minérale concentrée de l'invention permet d'obtenir une eau de source ou minérale concentrée ayant conservé l'équilibre ionique d'origine de l'eau de départ, avec un résidu sec qui reste faible. Par conséquent, la teneur en ions potentiellement interférents tels que les ions divalents et les bicarbonates, et notamment dans le cas de l'eau de Treignac, les ions calcium et magnésium et les bicarbonates, se retrouvent dans l'eau concentrée à une concentration particulièrement faible.

Globalement, le procédé est constitué par un passage simple en une étape de l'eau à concentrer dans un bloc d'osmose inverse sans d'autres étapes comme par exemple une étape d'électrodialyse. La membrane utilisée notamment dans les exemples est une membrane de type CSM (Customer Satisfaction Membrane) de référence RE 2540-TE. Le bloc d'osmose inverse comprend :

> Une cuve inox de 45 litres,
> Une pompe (CAT pumps 311), et
> Un support de membrane.

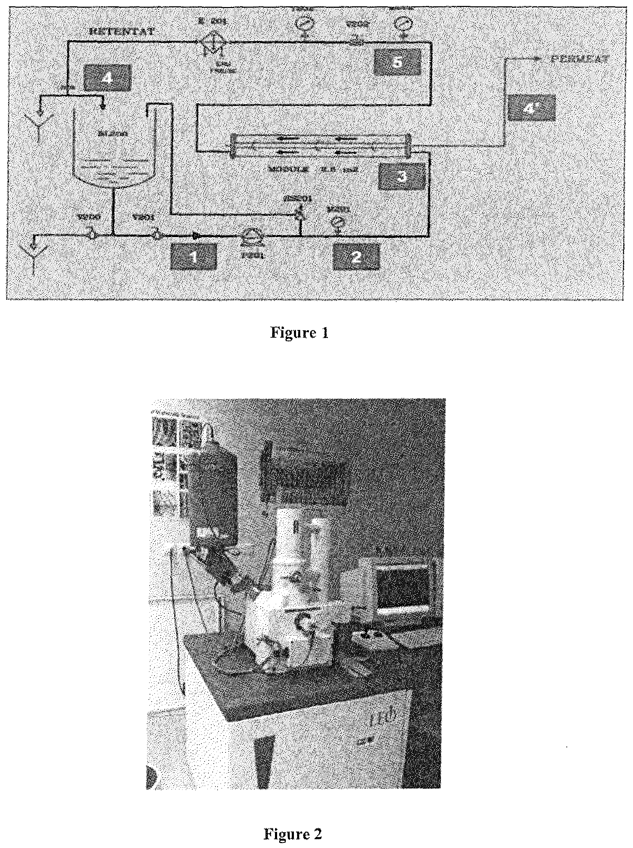

La pression au niveau de la membrane est de 10 bars et la température de 12°C. La figure 1 illustre le pilote utilisé dans les exemples pour concentrer l'eau de départ.

L'osmose inverse est un procédé connu de séparation en phase liquide qui permet l'élimination d'un solvant d'une solution par perméation sélective à travers une membrane sous l'action d'un gradient de pression. Le flux d'eau va de la solution concentrée en ions vers la solution diluée. L'écoulement de la solution à traiter est continu et tangentiel. La solution à traiter se divise en deux parties de concentrations différentes :

- Une partie qui passe à travers la membrane et qui est appelée perméat (solution très faiblement concentrée en ions),
- Une partie qui ne passe pas à travers la membrane et qui est appelée rétentat (ou concentrât) et qui contient les ions retenus par la membrane.

L'osmose inverse est une technologie membranaire de séparation bien connue de l'homme du métier.

La teneur en acide orthosilicique exprimée en $SiO_2$ de l'eau soumise à l'osmose inverse (appelé également l'eau de départ) est faible, de préférence, inférieure ou égale à 7 mg/l.

La faible minéralité de l'eau de départ permet sa concentration en une seule étape par osmose inverse. L'eau concentrée ainsi obtenue a une teneur en silice sensiblement accrue (d'un facteur d'au moins 8) tout en conservant l'équilibre ionique naturelle de l'eau minérale utilisée.

Dans le procédé de l'invention, il n'est pas nécessaire de soumettre le rétentat d'osmose inverse à d'autres procédés de séparation en phase liquide comme par exemple l'électrodialyse.

L'eau qui sera soumise à l'osmose inverse peut être une eau d'origine souterraine ou une eau de surface du moment où elle présente un résidu sec d'une eau ayant un résidu sec à 180°C inférieur à 50 mg/l et une teneur faible en acide orthosilicique (de préférence, inférieure ou égale à 7 mg/1). A ce titre, on peut citer par exemple, les eaux de table, les eaux de source et les eaux minérales naturelles.

Le procédé de l'invention peut mettre en oeuvre tout type d'eau, eau de table, eau de source ou eau minérale ayant une très faible minéralité et un pourcentage élevé en silice (de préférence supérieur à 20% du résidu sec).

De préférence, l'eau minérale soumise à l'osmose inverse est l'eau de Treignac qui est une eau souterraine présentant un résidu sec à 180°C de 20mg/l et une concentration en silice inférieure à 8mg/l en $SiO_2$ (soit 33% du résidu sec).

Au sens de l'invention, les eaux de table signifient une eau potable conforme aux normes nationales ou européennes. Par exemple, en Europe, l'eau potable est définie dans la Directive 98/83 CE. L'eau potable peut être l'eau du robinet.

Une eau de source peut être définie comme étant une eau d'origine souterraine, ayant bénéficié d'une protection contre la pollution, et n'ayant subi ni traitement chimique, ni adjonction. Elle est donc naturellement conforme et satisfait aux critères de potabilité notamment.

D'origine souterraine, à l'abri de toute pollution humaine, les eaux minérales naturelles se caractérisent par leur pureté originelle et par la stabilité de leur composition en minéraux et oligo-éléments, la conformité et la stabilité des analyses microbiologiques et la stabilité et la pérennité de la ressource. Les eaux minérales peuvent être classées selon leurs teneurs en minéraux :

> Eaux fortement minéralisées
> Eaux moyennement minéralisées
> Eaux faiblement minéralisées

Parmi ces eaux minérales on peut également distinguer :

➢ Les eaux sulfatées calciques,
➢ Les eaux bicarbonatées sodiques.

De préférence, l'eau de départ est une eau minérale naturelle. Plus préférentiellement, l'eau de départ est une eau minérale naturelle très peu minéralisée (un résidu sec à 180°C de moins de 50 mg/1) comme par exemple l'eau de Treignac (http://www.eau-treignac.com).

L'eau de source ou minérale concentrée de l'invention peut être utilisée dans les domaines divers comme par exemple, en cosmétique, en dermatologie, en pharmacie, en diététique et en nutrition notamment en tant que complément alimentaire.

Ainsi, l'invention a pour objet l'utilisation d'une eau concentrée selon l'invention, pour :

- régénérer et/ou renforcer la peau, les cheveux et les ongles ; et/ou
- hydrater et prévenir la déshydratation de la peau.

En effet, il a été démontré que l'eau concentrée de l'invention pénètre dans la peau et induit un effet hydratant important et particulièrement durable (essais faits chez l'homme par un laboratoire indépendant).

De plus, la dessiccation de l'eau induit une surconcentration des minéraux dont une polymérisation de l'acide orthosilicique (ratio molaire avec les cations très favorable) en l'absence d'ions ou de substances organiques potentiellement interférentes qui forme en surface de la peau un film imperméable protégeant de la déshydratation par évaporation. En dermatologie ce phénomène est connu comme la perte insensible en eau (PIE).

Le problème de déshydratation (manque d'humidité de la peau) peut toucher tout le monde, à tout âge et à degrés divers et particulièrement les personnes âgées. Outre les sensations de tiraillement, d'inconfort, de rugosité et de desquamation, une peau déshydratée peut rendre les rides plus apparentes et, d'une manière générale, donner un aspect peu esthétique à la peau dû à son manque d'élasticité.

Des facteurs comme les changements de temps (chaud/froid), la pollution, les changements hormonaux, le stress, l'usage fréquent de savons ou de shampoings, une exposition excessive au soleil ou au vent, etc. peuvent également sensibiliser et fragiliser la peau particulièrement chez les sujets ayant une peau dite « sensible ».

L'eau de source ou minérale concentrée de l'invention peut apporter une solution efficace et naturelle à ces problèmes grâce à notamment sa teneur élevée en silicium naturel. Par exemple, elle peut stimuler la formation des fibres de collagène et maintenir l'hydratation des tissus, ce qui est particulièrement indiqué pour tonifier, raffermir la peau et protéger les peaux sensibles (comme démontré chez 57 patients dans une étude dermatologique). Elle peut entrer dans la structure de la kératine pour densifier la chevelure et rendre les ongles moins cassants.

Grâce à sa propriété filmogène, l'eau de source ou minérale concentrée de l'invention peut former un film continu maintenant ainsi un état d'hydratation durable à la surface de la peau.

Dans le domaine pharmaceutique, grâce à sa teneur élevée en silicium naturelle et sa faible minéralisation, l'eau de source ou minérale concentrée de l'invention peut être destinée à être utilisée comme médicament et/ou complément alimentaire. Cela constitue un autre objet de l'invention.

Ainsi, l'eau de source ou minérale concentrée peut être destinée à être utilisée dans

- Le traitement et/ou la prévention des maladies neurodégénératives, en particulier la maladie d'Alzheimer ;
- Le traitement des peaux sensibles ;
- Le traitement et/ou la prévention des pathologies inflammatoires de la peau et, en général, des agressions cutanées ;

Un autre objet de l'invention consiste à proposer une composition diététique, cosmétique, dermatologique ou pharmaceutique ainsi qu'un complément nutritionnel comprenant, éventuellement en association avec tout excipient approprié, une eau de source ou minérale concentrée selon l'invention. Les excipients utilisés doivent être acceptables sur le plan pharmaceutique, biologique, cosmétique et alimentaire. Ces excipients peuvent être des conservateurs, des colorants, des arômes, des épaississants, des gélifiants, des stabilisants, des conservateurs, des parfums, etc.

Parmi les multiples utilisations de ces compositions et complément nutritionnel on peut citer, notamment :

- le ralentissement du vieillissement tissulaire grâce à une action antioxydante,
- la régénération et le renforcement de la peau, des cheveux, des ongles,
- le maintien d'une bonne hydratation des tissus notamment au niveau cutané,
- l'amélioration de la synthèse des fibres de collagènes et d'élastine,
- la préservation de l'élasticité des vaisseaux,
- la protection de la peau contre les pathologies inflammations.

L'invention a également pour objet un dispositif contenant une eau de source ou minérale concentrée selon l'invention ou une composition diététique, cosmétique, dermatologique ou pharmaceutique comprenant une eau concentrée selon l'invention. Un tel dispositif peut être choisi parmi les brumisateurs, les flacons vaporisateurs, etc.

L'eau de source ou minérale concentrée selon l'invention et une composition cosmétique, dermatologique ou pharmaceutique la comprenant peuvent ainsi être appliquées sous forme de fines particules liquides au moyen de brumisateurs, de flacons vaporisateurs, etc.

D'autres avantages et caractéristiques de la présente invention apparaîtront à la lecture des exemples ci-dessous donnés à titre illustratif et les figures.

La figure 1 représente le schéma du pilote utilisé pour la concentration de l'eau de départ soumise à une concentration où :

1 = arrivée de l'eau en tube inox
2= manomètre
3 = passage de l'eau dans la membrane
4= circulation du rétentat
5= manomètre de vérification de perte de charge.

La figure 2 représente le microscope électronique à balayage utilisé dans le laboratoire du Pr Devers, IUT de Chartres pour étudier les propriétés filmogènes de l'eau concentrée de l'invention.

La figure 3 représente l'image obtenue par microscope électronique à balayage montrant l'effet filmogène de l'eau concentrée de l'invention.

La figure 4 montre le film formé par l'eau minérale Evian.

La figure 5 représente l'effet de l'eau concentrée de l'invention sue les principaux signes des peaux sensibles, notamment le prurit et l'érythème.

La figure 6 représente les variations de l'effet d'hydratation cutanée de l'eau concentrée de l'invention exprimées en pourcentage.

## EXEMPLES

### EXEMPLE 1 : Procédé de Production de l'eau de source ou minérale concentrée selon l'invention par osmose inverse

Le procédé décrit dans cet exemple montre la préparation en une seule étape de l'eau concentrée de l'invention.

### I. Matériel et méthodes

*Principe de l'osmose inverse*

L'osmose inverse est une technologie membranaire de séparation. Toutefois, elle n'est pas un processus de filtration, comme en micro, nano ou ultra filtration, mais un processus de sélectivité par diffusion.

Une membrane semi-perméable est placée entre deux compartiments contenant des solutions de salinité différente. L'osmose est un phénomène naturel qui consiste en la migration de l'eau de la solution là moins concentrée vers la solution la plus concentrée (flux osmotique).

Lorsque l'on applique une pression supérieure à la pression osmotique sur la solution la plus concentrée, le flux d'eau est dirigé en sens inverse du flux osmotique, c'est-à-dire de la solution la plus concentrée vers la solution la moins concentrée : c'est le phénomène d'osmose inverse.

Une membrane d'osmose inverse est une membrane semi-perméable. Elle est constituée d'une couche dite « support » d'environ 50 $\mu$ d'épaisseur, et d'une couche dite « barrière » d'environ 0,2 $\mu$ d'épaisseur.

La solution à traiter est injectée dans la membrane par l'intermédiaire d'une pompe. Le concentrât est la solution qui est rejetée et qui n'a pas traversé la membrane. Elle est chargée en soluté (sels, matières organiques, virus, bactéries...).

Le perméat est la solution qui a traversé la membrane, qui est purifiée. Elle est recueillie en sortie de la membrane d'osmose.

*Caractéristique de la membrane*

Les membranes CSM (Customer satisfaction Membrane) sont des membranes polyamide en composite à couche mince (thin film composite, TFC). Elles sont constituées d'une couche support poreuse et d'une couche dense de film composite qui constitue la peau de la membrane réticulée qui est directement conçue in situ sur le support poreux, fabriqué en polysulfone. La référence du modèle utilisé est RE 2540-TE.

*Caractéristique du pilote*

Le pilote utilisé a été fabriqué par la société Techniques Industrielles Appliquées (TIA). Le modèle est le BL 200 procédé membranaire. La constitution du pilote est la suivante :

A) une cuve inox d'un volume de 45 L (diamètre de 31,5 cm et hauteur de 57 cm) ;
B) une pompe CAT Pumps 311 ;
C) un support pour mise en place de la membrane.

Les conditions opératoires pour la tenue de cet essai sont les suivantes :

- Pression : 10 bars
- Réfrigération : eau du robinet (température de 12 °C).

*Réalisation de l'essai*

Avant l'utilisation du pilote, ce dernier a été lavé à l'aide d'eau osmosée dont la conductivité était inférieure à 3 $\mu$S/cm. Ce lavage a également pour objectif de nettoyer la membrane avant son premier fonctionnement, notamment en éliminant la solution de bisulfite de sodium constituant le liquide de conservation. La durée du lavage est de 3 heures.
La cuve en inox est ensuite remplie d'eau de Treignac. Une fois cette cuve remplie, le système d'osmose inverse est mis en fonctionnement.
La quantité de liquide constituant le perméat est renouvelée par addition d'eau de Treignac en continu directement dans la cuve.
Le fonctionnement du pilote est maintenu jusqu'à l'obtention d'un volume de concentrât égal au huitième du volume initial d'eau de Treignac. Il s'agit d'une concentration par un facteur huit.
Le concentrât présent dans la cuve est ensuite récupérer au sein d'un bidon.
Le dosage de la silice est assuré par l'utilisation de test en cuve HACH Lange LCW 028.
La silice dissoute ou les silicates forment en solution acide avec le molybdate d'ammonium un acide silico-molybdique colorée jaune. L'addition d'un agent réducteur génère une coloration bleue.

Méthode :

- Pipeter 25,0 ml d'échantillon dans un bécher en plastique.
- Pipeter 1,0 ml de la solution A (LCW 028 A).
- Mélanger, attendre 3 minutes.
- Pipeter 1,0 ml de la solution B (LCW 028 B).
- Mélanger, attendre 3 minutes.
- Pipeter 1,0 ml de la solution C (LCW 028 C).
- Mélanger, laisser reposer pendant 25 minutes.
- Transférer dans la cuve rectangulaire de 50 mm.
- Bien nettoyer l'extérieur de la cuve et mesurer.

Le spectrophotomètre utilisé est un lecteur HACH Lange DR 2800.
Un dosage de la silice a été effectué sur le concentrât, une concentration de 55 mg/l de $SiO_2$ a été trouvée. Cette valeur correspond à un facteur de concentration de l'ordre de huit.
Des dosages de sodium et de calcium ont également été réalisés sur le concentrât ; les valeurs obtenues sont respectivement de 6,8 mg/l et 18,1 mg/1.
La concentration a donc bien été effectuée. Une partie de la silice peut avoir été retenue au sein de la membrane.
Le tableau ci-dessous montre la teneur en ions de l'eau de départ (eau de Treignac) ainsi que celle en acide orthosilicique exprimée en $SiO_2$.

| | $HCO_3^-$ (mg/l) | Résidu sec à 180°C | pH | Calcium (mg/l) | Acide orthosilicique (mg/l de $SiO_2$) | % Acide orthosilicique (% de $SiO_2$) |
|---|---|---|---|---|---|---|
| **Eau Treignac** | 3,3 | 20 | 5,6 | 0,9 | 6,6 | 33 |

(suite)

| | HCO$_3^-$ (mg/l) | Résidu sec à 180°C | pH | Calcium (mg/l) | Acide orthosilicique (mg/l de SiO$_2$) | % Acide orthosilicique (% de SiO$_2$) |
|---|---|---|---|---|---|---|
| Eau Treignac concentrée | 26,2 | 190 | 6,8 | 8,3 | 55 | 29 |

La très faible minéralité de l'eau de Treignac permet de la concentrer par osmose inverse, en une seule étape, sans modifier les rapports ioniques c'est-à-dire sans modifier les rapports concentration ionique/résidu sec, mais en augmentant sensiblement la concentration en silice (de 6,6 mg/l à 55 mg/l).

**EXEMPLE 2 : Propriétés filmogène de l'eau concentrée de l'invention**

Une étude a été réalisée dans le laboratoire du Pr Devers, IUT de Chartres par microscopie à balayage montré en figure 2.
Différentes eaux ont été déposées sur des lames de verre et laissées évaporer.
Ces études ont montré que l'eau concentrée de l'invention a un effet filmogène caractéristique : un film épais et homogène sans formation de cristaux comme montré en figure 3. La présence de cristaux peut avoir un effet délétère en particulier sur la peau.
La figure 4 montre, à titre comparatif, l'eau minérale Evian qui forme un film discontinu ou cassé et de nombreux cristaux de sel.

**EXEMPLE 3 : Propriétés apaisante de l'eau concentrée de l'invention**

Les propriétés apaisantes de l'eau concentrée selon l'invention (eau de Treignac concentrée) ont été démontrées par deux études effectuées chez 57 sujets.

**A/ Etude dermatologique sur 57 patients ayant des peaux sensibles**

Après brumisation de l'eau de Treignac concentrée sur la peau deux fois par jour pendant un mois, les résultats sont les suivants :

| | | Moyenne des scores | | |
|---|---|---|---|---|
| | | Avant | Après | Significativité statistique |
| Brulure | 13 | 1,69 | 0,65 | 0.007 |
| Tiraillements | 16 | 2,31 | 0,56 | 0.001 |
| Prurit | 10 | 1,70 | 0,35 | 0.007 |
| Intolerance aux agents topiques | 11 | 2,00 | 0,27 | 0.005 |

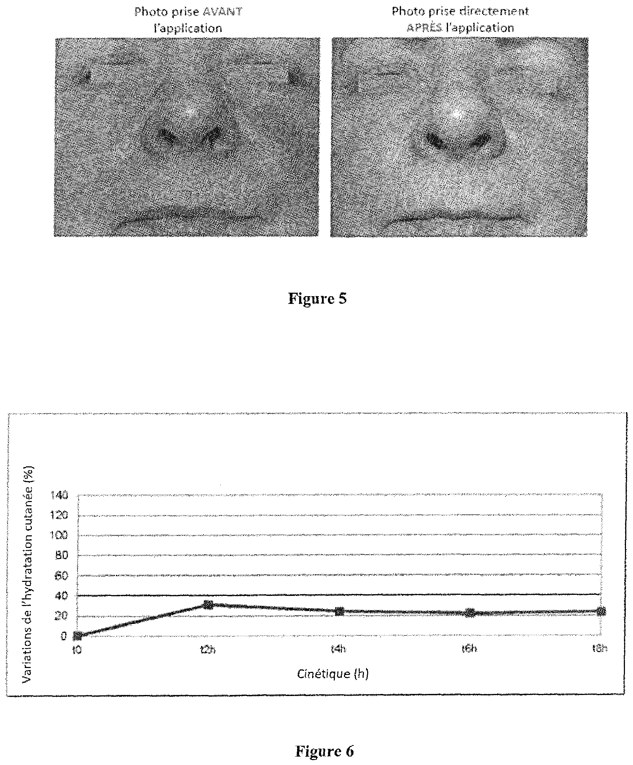

Ces résultats montrent un effet très significatif de l'eau de Treignac concentrée sur les principaux signes des peaux sensibles et particulièrement sur le prurit. D
Des effets comparables ont été observés pour l'érythème. Cet effet a été, chez certains patients, particulièrement rapide, au bout d'une heure comme montré en figure 5.

**B/ Test de picotement (Stinging Test en anglais) effectués par le laboratoire Dermscan**

Ce test qui est bien connu de l'homme du métier, est utilisé pour identifier les personnes ayant une réactivité cutanée particulière au niveau du visage, ce test permet d'évaluer des produits destinés aux peaux sensibles.
Ainsi, ce test permet de déterminer la sensibilité d'un sujet ou d'évaluer l'effet apaisant d'un produit. Dans le cas présent, le produit est l'eau concentrée de Treignac.
Les volontaires choisis pour leur réactivité cutanée ont appliqué l'eau concentrée de l'invention en conditions d'usage, Un stinging test a ensuite été réalisé pour évaluer l'action de l'eau concentrée de l'invention.
La sensation de picotement est cotée de 1 à 3 :

0 = rien
1 = léger picotement
2 = picotement modéré
3 = picotement intense.

Les résultats obtenus montrent qu'après 28 jours d'utilisation biquotidienne, l'eau concentrée de l'invention

- a été très bien toléré sur le plan cutané ;
- a présenté un effet apaisant important caractérisé par une diminution significative du score de réactivité cutanée de 56% en moyenne. Cet effet a été observé chez 86% des volontaire.

Par ailleurs, la majorité des volontaires a apprécié l'eau concentrée de l'invention pour ses caractéristiques et pour son efficacité.

Ainsi, l'eau concentrée de l'invention peut porter les mentions : « TESTE SOUS CONTROLE DERMATOLOGIQUE » et « CONVIENT AUX PEAUX SENSIBLES ».

**EXEMPLE 4 : Propriétés hydratantes de l'eau concentrée de l'invention**

L'évaluation de la propriété d'hydratation cutanée de l'eau concentrée de l'invention a été réalisée par le Laboratoire Demscan.

La technique utilisée est une technique couramment utilisée dans ce domaine qui est fondée sur les propriétés électriques de la peau. Les propriétés électriques de la peau sur lesquelles sont basées ces mesures sont l'impédance, la capacitance et la conductance.

La relation entre ces trois paramètres de la couche cornée, lorsqu'un courant alternatif est appliqué à la surface de la peau est représentée par l'équation suivante :

$$Z = \sqrt{R^2 + x^2}$$

avec :

- Z = Impédance,
- R = Résistance (R = 1/G conductance)
- X = Réactance (X = 1/(2fc$\pi$)

avec f = fréquence et c = capacitance)

La mesure de l'impédance réalisée avec un courant de basse fréquence traduit plutôt le taux d'hydratation des tissus vivants de la peau alors que la mesure de la conductance réalisé avec un courant de haute fréquence reflète plutôt l'hydratation du stratum comeum (SC).

Les tests d'hydratation cutanée induite par l'eau concentrée de l'invention ont été pratiquées à l'aide d'un coméomètre (COURAGE & KHAZAKA CM 825). Cet appareil analyse l'hydratation des couches les plus externes du stratum cornéum par mesure de la capacitance. La surface de la tête de mesure, en contact avec la peau, voit sa capacitance modifiée en fonction du degré d'humidité de la peau.

Les coméomètres sont simples d'utilisation. Toutefois, les mesures peuvent être influencées par différents paramètres sans que le taux réel d'hydratation cutanée en soit modifié. Ces paramètres peuvent être liés à la topographie cutanée, à la présence de poils ou encore à la pression exercée par l'appareil au moment de la mesure, engendrant une modification de l'aire de contact de la sonde avec la peau. Egalement, la température cutanée, la sécrétion de sueur et/ou de sébum, l'application de topiques riches en eau et/ou en ions jouent également un rôle dans la variation des mesures. Afin de réaliser des mesures d'hydratation cutanée précises et reproductibles, il est nécessaire de respecter des conditions expérimentales standardisées.

Cette étude a été réalisée sur 12 volontaires sains. Les mesures ont été effectuées avant l'application de l'eau concentrée puis 2, 4, 6 et 8 heures après l'application de l'eau concentrée sur la face interne des avant-bras. La réalisation des mesures a été faite après une acclimatation de 15 minutes et nettoyage des zones sur lesquelles a été appliquée l'eau concentrée.

Les résultats montrent que dans les conditions de cette étude, l'eau concentrée de l'invention (eau concentrée de Treignac) présente un effet hydratant significatif des couches superficielles de l'épiderme deux, quatre, six et huit heures après son application. Une augmentation du taux d'hydratation cutanée a été observée :

- 31% après 2 heures,
- 24% après 4 heures,
- 22% après 6 heures, et
- 23% après 8 heures.

L'analyse de la courbe montrée en figure 6 montre une hydratation maximale obtenue 2 heures après l'application de l'eau concentrée puis une légère diminution de l'hydratation aux temps 4, 6 et 8 heures sans jamais atteindre le niveau basal, montrant la rémanence de l'hydratation induite par application de l'eau concentrée de l'invention.

L'eau concentrée de l'invention présente donc un effet hydratant longue durée.

**Revendications**

1. Eau concentrée **caractérisée en ce qu'**elle présente un résidu sec d'au plus 195 mg/l à 180°C et une teneur en acide orthosilicique exprimée en $SiO_2$ d'au moins 40 mg/1.

2. Eau concentrée selon la revendication 1, **caractérisée en ce que** sa teneur en acide orthosilicique exprimée en $SiO_2$ est comprise entre 40 et 60 mg/l, bornes incluses.

3. Eau concentrée selon l'une des revendications 1 ou 2, **caractérisée en ce qu'**elle a un pH compris entre 6,0 et 7,5.

4. Eau concentrée selon l'une quelconque des revendications 1 à 3, **caractérisée en ce que** sa une teneur en bicarbonate ($HCO_3^-$) est inférieure ou égale à 30 mg/l, de préférence inférieure ou égale à 25 mg/l.

5. Procédé de production d'une eau concentrée selon l'une quelconque des revendications 1 à 4, **caractérisé en ce qu'**une eau minérale ou de source ayant un résidu sec à 180°C inférieur à 50 mg/l et une teneur en acide orthosilicique exprimée en $SiO_2$ inférieur à 7 mg/l est concentrée en une seule étape par osmose inverse de façon à obtenir une eau concentré présentant un résidu sec d'au plus 195 mg/l à 180°C et une teneur en acide orthosilicique exprimée en ($SiO_2$) d'au moins 40 mg/l

6. Utilisation d'une eau concentrée selon l'une quelconque des revendications 1 à 4, pour :

   - régénérer et/ou renforcer la peau, les cheveux et les ongles ; et/ou
   - hydrater et prévenir la déshydratation de la peau.

7. Eau concentrée selon l'une quelconque des revendications 1 à 4, destinée à être utilisée comme médicament.

8. Eau concentrée selon l'une quelconque des revendications 1 à 4, destinée à être utilisée dans

   - le traitement et/ou la prévention des maladies neurodégénératives, en particulier la maladie d'Alzheimer ;
   - le traitement des peaux sensibles ;
   - le traitement et/ou la prévention des pathologies inflammatoires de la peau.

9. Composition diététique, cosmétique, dermatologique ou pharmaceutique **caractérisée en ce qu'**elle comprend, éventuellement en association avec tout excipient approprié, une eau concentrée selon l'une quelconque des revendications 1 à 4.

10. Complément nutritionnel **caractérisé en ce qu'**il comprend, en association avec tout excipient approprié, une eau concentrée selon l'une quelconque des revendications 1 à 4.

11. Dispositif contenant une eau concentrée selon l'une quelconque des revendications 1 à 4 ou une composition diététique, cosmétique, dermatologique ou pharmaceutique selon la revendication 10.

12. Dispositif selon la revendication 11, **caractérisé en ce que** le dispositif est un système de brumisation choisi parmi les brumisateurs, les flacons vaporisateurs.

**Patentansprüche**

1. Konzentriertes Wasser, **dadurch gekennzeichnet, dass** es einen Trockenrückstand von höchstens 195 mg/l bei 180°C und einen Gehalt an Orthokieselsäure, ausgedrückt in $SiO_2$, von mindestens 40 mg/l aufweist.

2. Konzentriertes Wasser nach Anspruch 1, **dadurch gekennzeichnet, dass** sein Gehalt an Orthokieselsäure, ausgedrückt in $SiO_2$, 40 bis einschließlich 60 mg/l beträgt.

3. Konzentriertes Wasser nach Anspruch 1 oder 2, **dadurch gekennzeichnet, dass** es einen pH-Wert zwischen 6,0 und 7,5 aufweist.

4. Konzentriertes Wasser nach einem der Ansprüche 1 bis 3, **dadurch gekennzeichnet, dass** sein Gehalt an Bikarbonat ($HCO_3^-$) kleiner oder gleich 30 mg/l, vorzugsweise kleiner oder gleich 25 mg/l ist.

5. Verfahren zur Herstellung eines konzentrierten Wassers nach einem der Ansprüche 1 bis 4, **dadurch gekennzeichnet, dass** ein Mineral- oder Quellwasser, das einen Trockenrückstand bei 180°C von weniger als 50 mg/l und einen Gehalt an Orthokieselsäure, ausgedrückt in $SiO_2$, von weniger als 7 mg/l aufweist, in einem einzigen Schritt durch reverse Osmose derart konzentriert wird, dass ein konzentrierte Wasser erhalten wird, das einen Trockenrückstand von höchstens 195 mg/l bei 180°C und einen Gehalt an Orthokieselsäure, ausgedrückt in ($SiO_2$), von mindestens 40 mg/l aufweist.

6. Verwendung eines konzentrierten Wassers nach einem der Ansprüche 1 bis 4 zum:

    - Regenerieren und/oder Stärken der Haut, der Haare und der Nägel; und/oder
    - Hydrieren der Haut und Verhindern der Dehydration derselben.

7. Konzentriertes Wasser nach einem der Ansprüche 1 bis 4, das dazu vorgesehen ist, als Medikament verwendet zu werden.

8. Konzentriertes Wasser nach einem der Ansprüche 1 bis 4, das dazu vorgesehen ist, verwendet zu werden bei:

    - der Behandlung und/oder Vorbeugung neurodegenerativer Krankheiten, insbesondere der Alzheimer-Krankheit;
    - der Behandlung empfindlicher Hauttypen;
    - der Behandlung und/oder Vorbeugung entzündlicher Pathologien der Haut.

9. Diätetische, kosmetische, dermatologische oder pharmazeutische Zusammensetzung, **dadurch gekennzeichnet, dass** sie, eventuell in Verbindung mit einem beliebigen geeigneten Hilfsstoff, ein konzentriertes Wasser nach einem der Ansprüche 1 bis 4 umfasst.

10. Nahrungsergänzungsmittel, **dadurch gekennzeichnet, dass** es, in Verbindung mit einem beliebigen geeigneten Hilfsstoff, ein konzentriertes Wasser nach einem der Ansprüche 1 bis 4 umfasst.

11. Vorrichtung, die ein konzentriertes Wasser nach einem der Ansprüche 1 bis 4 oder eine diätetische, kosmetische, dermatologische oder pharmazeutische Zusammensetzung nach Anspruch 10 enthält.

12. Vorrichtung nach Anspruch 11, **dadurch gekennzeichnet, dass** die Vorrichtung ein Zerstäubungssystem ist, das ausgewählt ist aus den Zerstäubern, den Sprühflaschen.

**Claims**

1. A concentrated water, **characterised in that** it has a dry residue of at most 195 mg/l at 180°C and an orthosilicic acid content expressed as $SiO_2$ of at least 40 mg/l.

2. The concentrated water according to claim 1, **characterised in that** its orthosilicic acid content expressed as $SiO_2$ is between 40 and 60 mg/l, limits included.

3. The concentrated water according to one of claims 1 or 2, **characterised in that** it has a pH of between 6.0 and 7.5.

4. The concentrated water according to any one of claims 1 to 3, **characterised in that** its bicarbonate ($HCO_3^-$) content is less than or equal to 30 mg/l, preferably less than or equal to 25 mg/l.

5. A method for producing a concentrated water according to any one of claims 1 to 4, **characterized in that** a mineral or spring water having a dry residue at 180°C of less than 50 mg/l and an orthosilicic acid content expressed as $SiO_2$ of less than 7 mg/l is concentrated in a single step by reverse osmosis so as to obtain a concentrated water having a dry residue of at most 195 mg/l at 180°C and an orthosilicic acid content expressed as ($SiO_2$) of at least 40 mg/l.

6. A use of a concentrated water according to any one of claims 1 to 4, for:

   - regenerating and/or reinforcing the skin, hair and nails; and/or
   - hydrating the skin and preventing the dehydration of same.

7. The concentrated water according to any one of claims 1 to 4, intended for use as a drug.

8. The concentrated water according to any one of claims 1 to 4, intended for use in

   - the treatment and/or prevention of neurodegenerative diseases, in particular Alzheimer's disease;
   - the treatment of sensitive skin;
   - the treatment and/or prevention of inflammatory skin diseases.

9. A dietetic, cosmetic, dermatological or pharmaceutical composition, **characterized in that** it comprises, optionally in combination with any suitable excipient, a concentrated water according to any one of claims 1 to 4.

10. A nutritional supplement **characterised in that** it comprises, in association with any suitable excipient, a concentrated water according to any one of claims 1 to 4.

11. A device containing a concentrated water according to any one of claims 1 to 4 or a dietetic, cosmetic, dermatological or pharmaceutical composition according to claim 10.

12. The device according to claim 11, **characterised in that** the device is a misting system selected from misters and spray bottles.

Figure 1

Figure 2

Figure 3

Figure 4

Photo prise AVANT
l'application

Photo prise directement
APRÈS l'application

**Figure 5**

**Figure 6**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2456327 A